# EUROPÄISCHE PATENTANMELDUNG

(11) **EP 1 154 052 A1**
(43) Veröffentlichungstag der Anmeldung: **14.11.2001**
(21) Anmeldenummer: 01110995.6
(22) Anmeldetag: 07.05.2001
(51) Int. Cl.: D01G 9/08, A61F 13/15

(54) **Vorrichtung zum Bereitstellen eines von Flockenverklumpungen im wesentlichen freien Flocken-Luft-Gemisches sowie Verfahren zum Auflösen von Flockenverklumpungen**

(30) Priorität: 09.05.2000 DE 10022499
(71) Anmelder: Winkler + Dünnebier Aktiengesellschaft, 56562 Neuwied (DE)
(72) Erfinder: Geisen, Armin, 56581 Melsbach (DE); Haase, Sascha, 56564 Neuwied (DE); Mertgen, Ulrich, 56584 Meinborn (DE)
(74) Vertreter: Strych, Werner Maximilian Josef, Dr.

(57) **Zusammenfassung**

Es wird eine Vorrichtung bzw. ein Verfahren zum Bereitstellen eines Flocken-Luft-Gemisches, welches von einer Zerfaserungseinrichtung (3) durch eine Rohrleitung (7) einer Flockenlegeeinrichtung (4) zur Bildung eines Saugkissens (2) zugeführt wird, wobei im Verlauf der Rohrleitung (7) eine Auflöseeinrichtung (20) zum Auflösen von Flockenverklumpungen innerhalb des Flocken-Luft-Gemisches vorgesehen ist, beschrieben, bei welcher bzw. bei welchem ein möglichst hoher Wirkungsgrad bei der Auflösung der Flockenverklumpungen erreicht werden soll, wobei dieser Wirkungsgrad möglichst unabhängig vom Durchsatz des Flocken-Luft-Gemisches durch die Rohrleitung (7) zwischen Zerfaserungseinrichtung (3) und Flockenlegeeinrichtung (4) sein soll. Zur Lösung dieser Aufgabe wird vorgeschlagen, die Auflöseeinrichtung (20) als pneumatische Auflöseeinrichtung (20) auszubilden, welche die Flockenverklumpungen auflöst, indem sie das Flocken-Luft-Gemisch samt der darin enthaltenen Flockenverklumpungen derart beschleunigt, dass die Flockenverklumpungen zerplatzen oder zerreißen.

## Beschreibung

Die vorliegende Erfindung betrifft eine Vorrichtung zum Bereitstellen eines Flocken-Luft-Gemisches gemäß dem Oberbegriff des Anspruchs 1 sowie ein Verfahren zum Auflösen von Flockenverklumpungen gemäß dem Oberbegriff des Anspruchs 11.

Bei Maschinen zur Herstellung von saugfähigen Kissen für Damenbinden, Höschenwindeln und ähnliches ist es üblich, auf einer Flockenlegeeinrichtung ein Saugkissen oder Flockenkern aus einem hydrophilen Flockengemisch zu bilden. Die hierzu benötigten Flocken werden der Flockenlegeeinrichtung als Flocken-Luft-Gemisch von einer Zerfaserungseinrichtung kommend durch eine Rohrleitung zugeführt.

Aus Umwelt- und Lärmschutzgründen ist es üblich geworden, die Zerfaserungseinrichtung räumlich getrennt von der Flockenlegeeinrichtung anzuordnen und zur Überwindung der dadurch größeren Förderdistanz ein Gebläse oder Ventilator in die Rohrleitung zu integrieren. In der längeren Rohrleitung kommt es durch Rohrreibung, Strömungsturbulenzen sowie statische Aufladung zu Flockenverdichtungen bzw. Flockenverklumpungen innerhalb des Flocken-Luft-Gemisches. Dies verhindert den gewünschten homogenen Flockenaufbau des Saugkissens und beeinträchtigt darüberhinaus die mechanische Stabilität des Saugkissens, da Verklumpungen nur in das Flockennetzwerk des Saugkissens eingelegt, nicht aber eingebunden werden.

Es ist bekannt, zur Auflösung der Flockenverklumpungen nahe der Flockenlegeeinrichtung einen zusätzlichen, angetriebenen Mühlenrotor in die die Flocken zuführende Rohrleitung zu integrieren. Diese Technik ist beispielsweise in den Dokumenten US 1,834,309, US 2,940,133, US 2,940,134, US 2,940,135, US 3,886,629 sowie US 4,375,447 beschrieben. Nachteilig ist bei diesen bekannten Lösungen, dass sie allesamt teuer sind und mit erheblichem Raumbedarf verbunden sind. Die bekannten Mühlenrotoren benötigen einen Drehantrieb, dessen Drehzahl in Abhängigkeit des Durchsatzes des Flocken-Luft-Gemisches angepaßt werden muß. Darüberhinaus erzeugen die Mühlenrotoren unerwünschte Luftverwirbelungen, die einer gleichmäßigen Ausbildung des Saugkissens entgegenwirken.

Als Alternative zu einem Mühlenrotor in der die Flocken zuführenden Rohrleitung wird in der EP 0 515 939 A1 vorgeschlagen, eine mechanische Pralleinrichtung in der Rohrleitung vorzusehen. Diese bekannte Pralleinrichtung besteht aus mehreren Prallstäben, welche in den Querschnitt der das Flocken-Luft-Gemisch transportierenden Rohrleitung hineinragen. Diejenigen in dem Flocken-Luft-Gemisch enthaltenen Flockenverklumpungen, welche beim Transport von der Zerfaserungseinrichtung zu dem Flockenleger gegen die Prallstäbe prallen, werden aufgrund dieser mechanischen Kollision weitestgehend aufgelöst. Als nachteilig hat sich bei der bekannten Pralleinrichtung erwiesen, dass einerseits durchaus noch nennenswerte Mengen von Flockenverklumpungen nicht mit den Prallstäben kollidieren und somit nicht aufgelöst werden und andererseits der Wirkungsgrad der Flockenklumpungsauflösung vom Durchsatz des Flocken-Luft-Gemisches durch die Rohrleitung abhängig ist, da von diesem die Geschwindigkeit bestimmt wird, mit welcher die Flockenverklumpungen auf die Prallstäbe auftreffen.

Es ist daher die Aufgabe der vorliegenden Erfindung, eine Vorrichtung zum Bereitstellen eines von Flockenverklumpungen im wesentlichen freien Flocken-Luft-Gemisches bzw. ein Verfahren zum Auflösen von Flockenverklumpungen zu schaffen, bei welcher bzw. bei welchem ein möglichst hoher Wirkungsgrad bei der Auflösung der Flockenverklumpungen erreicht wird, wobei dieser Wirkungsgrad unabhängig vom Durchsatz des Flocken-Luft-Gemisches durch die Rohrleitung zwischen Zerfaserungseinrichtung und Flockenlegeeinrichtung sein soll.

Diese Aufgabe wird mit einer Vorrichtung mit den Merkmalen des Anspruchs 1 bzw. mit einem Verfahren mit den Merkmalen des Anspruchs 11 gelöst. Weitere Ausgestaltungen der Erfindung ergeben sich aus den Unteransprüchen.

Erfindungsgemäß wird eine Vorrichtung zum Bereitstellen eines Flocken-Luft-Gemisches, welches durch eine Rohrleitung einer Flockenlegeeinrichtung zur Bildung eines Saugkissens zugeführt wird, wobei im Verlauf der Rohrleitung eine Auflöseeinrichtung zum Auflösen von Flockenverklumpungen innerhalb des Flocken-Luft-Gemisches vorgesehen ist, vorgeschlagen, welche dadurch gekennzeichnet ist, dass die Auflöseeinrichtung von einer pneumatischen Auflöseeinrichtung gebildet wird, welche die Flockenverklumpungen auflöst, indem sie das Flocken-Luft-Gemisch samt der darin enthaltenen Flockenverklumpungen derart beschleunigt, dass die Flockenverklumpungen zerplatzen oder zerreißen.

Überraschenderweise hat sich herausgestellt, dass Flockenverklumpungen, welche aus verdichteten Zelluloseflocken bzw. -fasern bestehen, aufgelöst werden können, wenn sie plötzlich ruck-, stoß- oder sprungartig mit Hilfe eines gerichteten Fluidstroms, insbesondere Luftstroms, hoher kinetischer Energie beschleunigt werden. Dabei treten Beschleunigungs- und/oder Fluidreibungskräfte auf, welche die mechanischen und/oder elektrostatischen Adhäsionskräfte, die zwischen den eine Flockenverklumpung bildenden Flocken wirken, ohne weiteres überwinden. Darüber hinaus erzeugt die erfindungsgemäße, pneumatische Auflöseeinrichtung vorzugsweise turbulente Strömungen, deren richtungs- sowie betragsmäßig willkürlich wechselnde Beschleunigungen dazu führen, dass die Flockenverklumpungen zerfallen.

Je nachdem wie groß die eine Flockenverklumpung zusammenhaltenden Kräfte sind, erfolgt die Auflösung der Flockenverklumpung früher oder später. Eine weniger fest zusammengehaltene Flockenverklumpung kann bereits zu Beginn der Beschleunigungsphase auseinandergerissen werden während eine fester zusammengehaltene Flockenverklumpung unter Umständen erst nach Erreichen eines hinreichenden Turbulenzgrades der Strömung des Flocken-Luft-Gemisches zersetzt werden kann. In jedem Fall wird erfindungsgemäß erreicht, dass im Gegensatz zu den bekannten Pralleinrichtungen sämtliche im Flocken-Luft-Gemisch enthaltenen Flockenverklumpungen von den Beschleunigungskräften erfasst werden.

Eine pneumatische Auflöseeinrichtung kann erfindungsgemäß im Endbereich der den Flocken-Luft-Gemisch-Strom liefernden Rohrleitung angeordnet werden. Vorzugsweise wird die Auflöseeinrichtung direkt in die Mündung der Rohrleitung in die Flockenlegeeinrichtung eingesetzt. Die Flockenlegeeinrichtung umfaßt ein sich drehendes Flockenformrad sowie einen ortsfesten Flockenkasten, in welchen die Rohrleitung über die pneumatische Auflöseeinrichtung mündet. Aufgrund des gerichteten Strahls von im wesentlichen verklumpungsfreiem Flocken-Luft-Gemisch, welcher von der pneumatischen Auflöseeinrichtung erzeugt wird, ist es im Gegensatz zum Stand der Technik sowie in vorteilhafter Weise möglich, diesen Strahl gezielt in eine bestimmte Raumrichtung zu richten. Die in der Umfangsfläche. des Flockenformrades befindlichen Formvertiefungen bzw. Mulden zum Ausbilden der Saugkissen können dadurch effektiver mit Flocke befüllt werden.

Erfindungsgemäß kann die pneumatische Auflöseeinrichtung von einer von dem Flocken-Luft-Gemisch umströmten Düse gebildet werden, welche etwa im mittleren Bereich des Querschnitts der Rohrleitung zwischen Zerfaserungseinrichtung und Flockenlegeeinrichtung angeordnet ist. Bei dieser Variante der Auflöseeinrichtung ist es allerdings erforderlich, die mit hohem Druck aus der Düse auszubringende Druckluft ebenso in den mittleren Bereich des Rohrleitungsquerschnitts zu bringen. Dies hat zur Folge, dass wenigstens eine entsprechende Pneumatikleitung in den mittleren Bereich des Rohrleitungsquerschnitts geführt werden muß, was bedeutet, dass die Pneumatikleitungen im Strömungsweg des Flocken-Luft-Gemisches stehen und somit zusätzlich zu der pneumatischen Auflösung der Flockenverklumpungen eine mechanische Auflösungswirkung im Sinne der bekannten Prallstäbe bewirken.

Vorzugsweise wird die pneumatische Auflöseeinrichtung in Form einer rohrstückartigen Injektordüse gewählt, welche selbst von dem Flocken-Luft-Gemisch durchströmt wird. Über einen, vorzugsweise mehrere Zuführdurchlässe in Form von Zuführkanälen wird dieser Injektordüse ein unter hohem Druck stehendes Druckfluid, insbesondere Druckluft, zugeführt. Dieses Druckfluid kann entweder von einer extra hierfür vorgesehenen Druckfluidquelle oder von einer ohnehin für andere Systeme der hier in Rede stehenden Maschinen bereits vorhandenen Druckfluidquelle stammen. Die vorzugsweise mehreren Zuführkanäle können sich erfindungsgemäß im äußeren Umfangsbereich desjenigen Raumes der Injektordüse befinden, der von dem Flocken-Luft-Gemisch durchströmt wird.

Die pneumatische Auflöseeinrichtung bzw. die Injektordüse wirken fluidmechanisch wie ein Fluid- bzw. Luftstromverstärker. Auf ihrer Eintritts- bzw. Ansaugseite entsteht ein Unterdruck während auf ihrer Austritts- bzw. Ausblasseite ein Überdruck entsteht. Die Auflöseeinrichtung bzw. Injektordüse kann daher wie ein am Ende der den Flocken-Luft-Strom liefernden Rohrleitung angeordnetes Sauggebläse wirken, so dass je nach Gesamtlänge der Rohrleitung in vorteilhafter Weise auf ein weiteres Gebläse in der Flockenförderstrecke verzichtet werden kann.

Erfindungsgemäß wird des weiteren ein Verfahren zum Auflösen von Flockenverklumpungen innerhalb eines Flocken-Luft-Gemisches, welches durch eine Rohrleitung einer Flockenlegeeinrichtung zur Bildung eines Saugkissens zugeführt wird, vorgeschlagen, welches dadurch gekennzeichnet ist, dass das Flocken-Luft-Gemisch samt der darin enthaltenen Flockenverklumpungen vor dem Ausbilden des Saugkissens in der Flockenlegeeinrichtung mittels einer pneumatischen Auflöseeinrichtung derart beschleunigt wird, dass die Flockenverklumpungen zerplatzen oder zerreißen. Es hat sich als besonders vorteilhaft herausgestellt, das Flocken-Luft-Gemisch auf Überschallgeschwindigkeit, vorzugsweise auf bis zu 2-fache Schallgeschwindigkeit zu beschleunigen. Derart hohe Geschwindigkeiten gewährleisten, dass in der Auflöseeinrichtung bzw. Injektordüse turbulente Strömungen erreicht werden und das Flocken-Luft-Gemisch hinreichend ruck-, stoß- oder sprungartig beschleunigt wird.

Nachfolgend wird eine Ausführungsform der vorliegenden Erfindung beispielhaft anhand der beigefügten Zeichnungen beschrieben. Es zeigen:
- Fig. 1:: eine schematische Seitenansicht einer erfindungsgemäßen Vorrichtung zum Bereitstellen eines Flocken-Luft-Gemisches für die Herstellung von Saugkissen;
- Fig. 2:: eine pneumatische Auflöseeinrichtung in Form einer Injektordüse;

- Fig. 3: eine Ansicht der Injektordüse gemäß Fig. 2 von oben; und
- Fig. 4:: den Schnitt A-A gemäß Fig. 3.

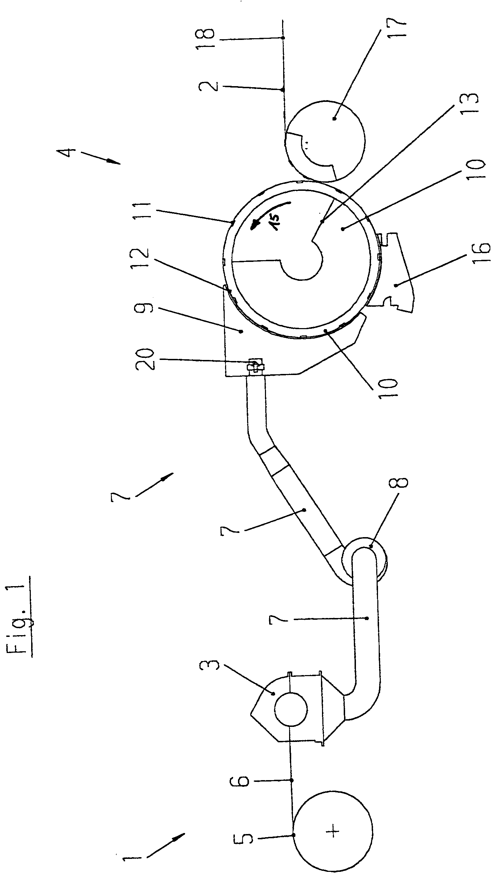

Fig. 1 zeigt eine erfindungsgemäße Vorrichtung zum Bereitstellen eines von Flockenverklumpungen im wesentlichen freien Flocken-Luft-Gemisches, aus welchem Flockenkerne bzw. Saugkissen 2 für Damenbinden, Höschenwindeln oder ähnliche Hygieneartikel hergestellt werden. Eine Mühle oder Zerfaserungseinrichtung 3 wird von einer Vorratsrolle 5 aus mit einer Papp- bzw. Zellstoffbahn 6 beschickt. Die Zellstoffbahn 6 wird in der Zerfaserungseinrichtung 3 zu Zellstoff-Flocken aufgelöst. Alternativ könnten die Zellstoff-Flocken auch auf sonstige Weise zur Verfügung gestellt werden, beispielsweise bereits als fertige Zellstoff-Flocken vorliegen. Diese Flocken werden mit Tragluft versetzt und als Flocken-Luft-Gemisch durch eine Rohrleitung 7 zu einer Flockenlegeeinrichtung 4 transportiert. Je nach Länge der Rohrleitung 7 kann dabei ein Gebläse bzw. Ventilator 8 zur Unterstützung des Tragluftstromes im Verlauf der Rohrleitung 7 vorgesehen werden.

Die Flockenlegeeinrichtung 4 umfaßt im wesentlichen ein Flockenformrad 10 sowie einen hohlen Flockenkasten 9, der das Flockenformrad 10 in einem vorgegebenen Umfangsbereich überdeckt und dort zum Flockenformrad 10 hin offen ist. Das Flockenformrad 10 dreht sich gemäß Drehrichtung 15 entgegen dem Uhrzeigersinn. Das Flockenformrad 10 weist in seinem Mantel bzw. in seiner äußeren Umfangsfläche Mulden oder Formvertiefungen 11 auf, welche mit siebartigen Böden 12 versehen sind. Ein ortsfester, d.h. sich nicht mit dem Flockenformrad 10 mitdrehender Saugkasten 13 ermöglicht es, Flocken-Luft-Gemisch in demjenigen Umfangsbereich des Flockenformrades 10, welcher von dem Flockenkasten 9 überdeckt wird, durch die siebartigen Böden 12 in die Formvertiefungen 11 zu saugen. Dadurch entstehen in denjenigen Formvertiefungen 11, welche sich in dem vom Flockenkasten 9 überdeckten Bereich befinden, die gewünschten Flockenkerne bzw. Saugkissen 2.

In Drehrichtung 15 vor dem Flockenformrad 10 ist eine Abbürsteinrichtung 16 angeordnet, welche das über die äußere Umfangsfläche aus den Formvertiefungen 11 hinausragende Flockenmaterial entfernt und über eine nicht dargestellte Rohrleitung zur Zerfaserungseinrichtung 3 zurückführt. Anschließend werden die auf diese Weise bündig gebürsteten Saugkissen mittels einer Saugwalze 17 aus den Formvertiefungen 11 entnommen und auf ein Transportband 18 gelegt, welches sie einer nicht gezeigten Maschine zur Herstellung von Damenbinden, Slipeinlagen, Höschenwindeln oder ähnliches zuführt.

Wie in Fig. 1 zu erkennen ist, mündet die Rohrleitung 7 unmittelbar in den Flockenkasten 9, wobei sich die bei der gezeigten Ausführungsform als Injektordüse 20 ausgebildete Auflöseeinrichtung bereits vollständig innerhalb des Flockenkastens 9 befindet. Auf diese Weise ist die Bewegungsfreiheit der Injektordüse 20 nicht durch die den Flockenkasten 9 in Fig. 1 nach links begrenzende Flockenkastenwandung eingeschränkt, so dass die Austrittsrichtung des die Injektordüse 20 verlassenden Flocken-Luft-Gemisch-Strahls problemlos auf eine bestimmte Raumrichtung eingestellt werden kann. Alternativ kann die Injektordüse 20 auch außerhalb des Flockenkastens 9 angeordnet werden.

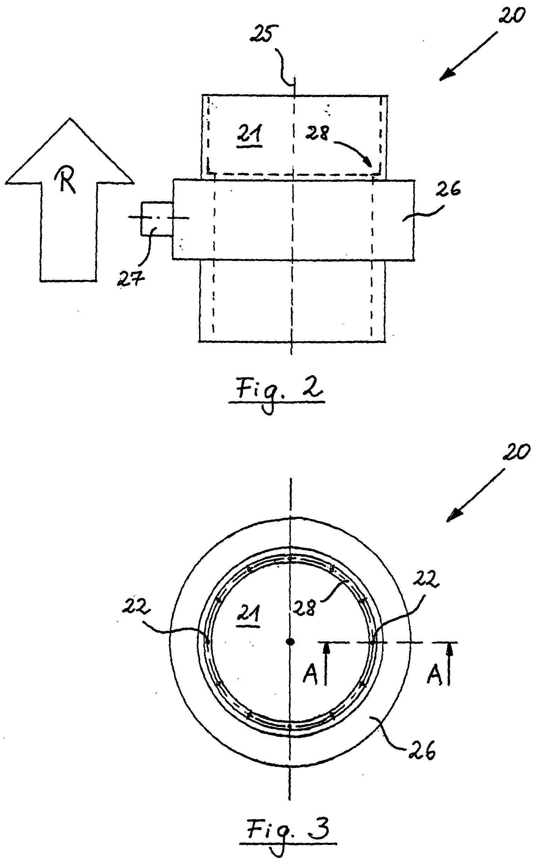

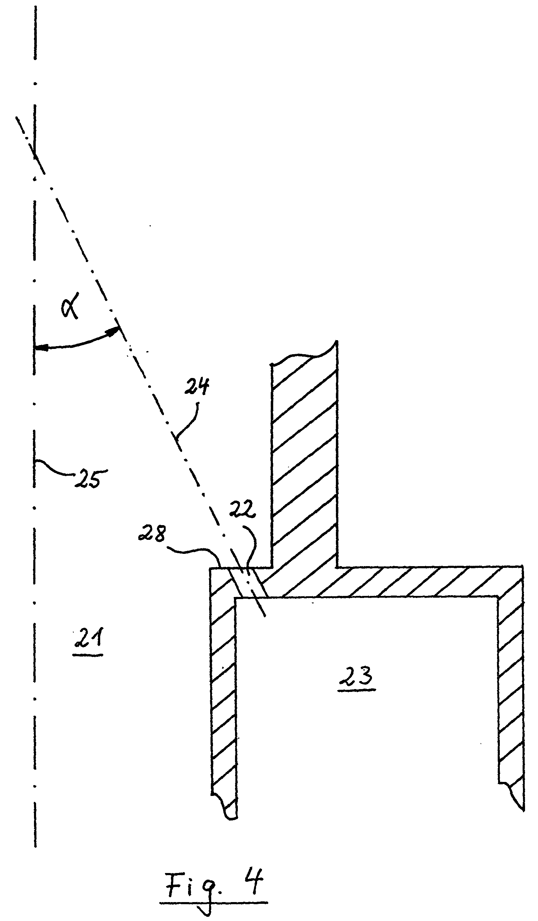

In den Fig. 2 und 3 ist die als pneumatische Auflöseeinrichtung fungierende Injektordüse 20 gezeigt. Sie besteht aus einem verhältnismäßig kurzen Rohrstück, welches in das stromabwärtige Ende der Rohrleitung 7 eingesetzt wird. Die Injektordüse 20 weist in ihrem in Längsrichtung mittleren Bereich einen Wulst 26 auf, in welchem ein vollständig umlaufender Ringkanal 23 ausgebildet ist, wie in der Schnittdarstellung gemäß Fig. 4 zu erkennen ist. Der Ringkanal 23 steht über den Anschlußstutzen 27 in Strömungsverbindung mit einer nicht gezeigten Pneumatik-bzw. Druckluftquelle, welche eigens für die Injektordüse 20 vorgesehen werden kann oder bereits für andere Systeme der jeweiligen Maschine vorhanden ist.

In der in Fig. 2 gezeigten Darstellung durchströmt das Flocken-Luft-Gemisch gemäß Pfeil R die Injektordüse 20 von unten nach oben. Dabei durchströmt es insbesondere den Durchströmungsraum 21, welcher bei der gezeigten Ausführungsform einen zylindrischen Querschnitt aufweist, alternativ jedoch auch mit einem in Durchströmungsrichtung R zunehmenden Querschnitt versehen sein kann und dadurch als Diffusor wirkt. Wie den Fig. 2 und 3 zu entnehmen ist, weist der Durchströmungsraum 21 einen größeren Innendurchmesser auf als der restliche, in Fig. 2 darunter liegende Raum der Injektordüse 20. Dadurch entsteht auf Höhe der unteren Begrenzungsebene des Durchströmungsraums 21 ein kreisringförmiger Absatz 28, wie in der Ansicht gemäß Fig. 3 dargestellt.

Wie am besten in den Fig. 3 und 4 zu sehen ist, münden in die kreisringförmige Fläche des Absatzes 28 bei der gezeigten Ausführungsform insgesamt zwölf als Zuführdurchlässe fungierende Zuführkanäle 22, welche in gleichmäßigen Winkelabständen über den Verlauf des Absatzes 28 verteilt sind. Alternativ ist auch denkbar, weniger oder mehr als zwölf Zuführkanäle vorzusehen oder die Zuführkanäle in ungleichmäßigen Winkelabständen anzuordnen, um bei Bedarf spezielle Strömungsprofile über den Querschnitt der Injektordüse 20 zu erhalten.

Wie in Fig. 4 gezeigt, stehen die Zuführkanäle 22 in Strömungsverbindung mit dem Ringkanal 23 und daher auch mit der an dem Anschlußstutzen 27 angeschlossenen Druckluftquelle.

Gemäß Fig. 4 ist die Achse 24 des Zuführkanals 22 um den Neigungswinkel α gegenüber der Längs- bzw. Symmetrieachse 25 der Injektordüse 20 geneigt. Dadurch wird bewirkt, dass die Druckluft nicht nur axial in Durchströmungsrichtung R, sondern auch mit einer radial nach innen auf die Längsachse 25 gerichteten Geschnwindigkeitskomponente zugeführt wird. Hierdurch kann zusätzlich zu den die Flockenverklumpungen auflösenden Beschleunigungskräften noch ein weiterer, die Flockenverklumpungsauflösung unterstützender Effekt bewirkt werden, der darin besteht, dass vorhandene Flockenverklumpungen aus den radial äußeren Bereichen des Strahlquerschnitts radial nach innen geblasen werden und dadurch unter Umständen miteinander kollidieren sowie dabei zerrissen bzw. aufgelöst werden. Der Neigungswinkel α kann erfindungsgemäß 50° oder weniger betragen, wobei für ihn vorzugsweise die Ungleichung 5° ≤ α ≤ 30° gilt. Alternativ kann der Neigungswinkel α auch 0° betragen, was bedeutet, dass die Achse 24 der Zuführkanäle 22 parallel zur Längsachse 25 der Injektordüse 20 verläuft.

Anstatt mehrerer Zuführdurchlässe 22 in Form von Bohrungskanälen kann zwischen Ringkanal 23 und Durchströmungsraum 21 auch ein einziger Zuführdurchlass in Form eines umlaufenden Ringspaltkanals vorgesehen werden. In diesem Fall steht der zuzuführenden Druckluft eine größere Querschnittsfläche zum Einströmen in den Durchströmungsraum 21 zur Verfügung, so dass dem Durchströmungsraum 21 ein größerer und räumlich besser verteilter Volumenstrom an Druckluft zugeführt werden kann und somit eine höhere Effektivität bei der Auflösung der Flockenverklumpungen realisierbar ist.

## Patentansprüche

1. Vorrichtung zum Bereitstellen eines Flocken-Luft-Gemisches, welches durch eine Rohrleitung (7) einer Flockenlegeeinrichtung (4) zur Bildung eines Saugkissens (2) zugeführt wird, wobei im Verlauf der Rohrleitung (7) eine Auflöseeinrichtung (20) zum Auflösen von Flockenverklumpungen innerhalb des Flocken-Luft-Gemisches vorgesehen ist,
**dadurch gekennzeichnet, dass**
die Auflöseeinrichtung eine pneumatische Auflöseeinrichtung (20) ist, welche die Flockenverklumpungen auflöst, indem sie das Flocken-Luft-Gemisch samt der darin enthaltenen Flockenverklumpungen derart beschleunigt, dass die Flockenverklumpungen zerplatzen oder zerreißen.

2. Vorrichtung nach Anspruch 1,
**dadurch gekennzeichnet, dass**
die Auflöseeinrichtung (20) im stromabwärtigen Endbereich der Rohrleitung (7) angeordnet ist.

3. Vorrichtung nach Anspruch 1 oder 2,
**dadurch gekennzeichnet, dass**
die Auflöseeinrichtung eine im wesentlichen im mittleren Bereich des Querschnitts der Rohrleitung (7) angeordnete Düse ist, welche von dem Flocken-Luft-Gemisch umströmt wird und wenigstens einen Düsenkanal aufweist, durch welchen Druckfluid zum Beschleunigen der Flockenverklumpungen von einer Druckfluidquelle zuführbar ist.

4. Vorrichtung nach Anspruch 1 oder 2,
**dadurch gekennzeichnet, dass**
die Auflöseeinrichtung von einer rohrstückartigen Injektordüse (20) gebildet wird, welche einen Durchströmungsraum (21), der von dem Flocken-Luft-Gemisch durchströmt wird, aufweist und mit wenigstens einem Zuführdurchlaß (22) versehen ist, welcher sich im äußeren Umfangsbereich des Durchströmungsraum (21) befindet und durch welchen dem Durchströmungsraum (21) Druckfluid zum Beschleunigen der Flockenverklumpungen von einer Druckfluidquelle zuführbar ist.

5. Vorrichtung nach Anspruch 4,
**dadurch gekennzeichnet, dass**
der wenigstens eine Zuführdurchlaß (22) mit einem Ringkanal (23) in Strömungsverbindung steht, der mit dem Druckfluid versorgbar ist.

6. Vorrichtung nach Anspruch 5,
**dadurch gekennzeichnet, dass**
mehrere Zuführdurchlässe in Form von Zuführkanälen (22) vorgesehen sind, welche in gleichmäßigen Winkelabständen in dem äußeren Umfangsbereich des Durchströmungsraums (21) angeordnet sind.

7. Vorrichtung nach einem der Ansprüche 4, 5 oder 6,
**dadurch gekennzeichnet, dass**
die Achse bzw. die Achsen (24) des Zuführdurchlasses bzw. der Zuführdurchlässe (22) parallel zu der Längsachse (25) der Injektordüse (20) verläuft bzw. verlaufen, so dass das Druckfluid dem Durchströmungsraum (21) im wesentlichen axial in Durchströmungsrichtung (R) des Flocken-Luft-Gemisches zugeführt wird.

8. Vorrichtung nach einem der Ansprüche 4, 5 oder 6,
**dadurch gekennzeichnet, dass**
die Achse bzw. Achsen (24) des Zuführdurchlasses bzw. der Zuführdurchlässe (22) um einen Neigungswinkel (α) geneigt zu der Längsachse (25) der Injektordüse (20) verläuft bzw. verlaufen, so dass das Druckfluid dem Durchströmungsraum (21) sowohl mit axialer als auch mit radialer Strömungskomponente zugeführt wird.

9. Vorrichtung nach Anspruch 8,
**dadurch gekennzeichnet, dass**
für den Neigungswinkel (α) die Ungleichung 0 ≤ α ≤ 50° gilt.

10. Vorrichtung nach Anspruch 9,
**dadurch gekennzeichnet, dass**
für den Neigungswinkel (α) die Ungleichung 5°≤ α ≤ 30° gilt.

11. Verfahren zum Auflösen von Flockenverklumpungen innerhalb eines Flocken-Luft-Gemisches, welches durch eine Rohrleitung (7) einer Flockenlegeeinrichtung (4) zur Bildung eines Saugkissens (2) zugeführt wird,
**dadurch gekennzeichnet, dass**
das Flocken-Luft-Gemisch samt der darin enthaltenen Flockenverklumpungen vor dem Ausbilden des Saugkissens (2) in der Flockenlegeeinrichtung (4) mittels einer pneumatischen Auflöseeinrichtung (20) derart beschleunigt wird, dass die Flockenverklumpungen zerplatzen oder zerreißen.

12. Verfahren nach Anspruch 11,
**dadurch gekennzeichnet, dass**
das Flocken-Luft-Gemisch auf Überschallgeschwindigkeit beschleunigt wird.

13. Verfahren nach Anspruch 12,
**dadurch gekennzeichnet, dass**
das Flocken-Luft-Gemisch auf etwa 2-fache Schallgeschwindigkeit beschleunigt wird.
